# EUROPEAN PATENT APPLICATION

(11) **EP 3 531 428 A1**
(43) Date of publication of application: **28.08.2019**
(21) Application number: 18158142.2
(22) Date of filing: 22.02.2018
(51) Int. Cl.: G16H 50/50, G06T 15/00, G06M 11/00

(54) **METHOD FOR USE IN TISSUE ENGINEERING**

(71) Applicant: Cell Therapy Catapult Limited, London, Greater London SE1 9RT (GB)
(72) Inventor: BARADEZ, Marc-Olivier, London, Greater London SE1 9RT (GB)
(74) Representative: Fennell, Gareth Charles

(57) **Abstract**

A computer-implemented method for use in determining the distribution of objects in a tubular structure is provided. The method comprises: receiving a representation of a cross-sectional slice of the tubular structure; based on the representation, determining a number of the objects in each of a plurality of circular sectors of the cross-sectional slice; and based on the number of objects in each of the plurality of circular sectors, outputting an indication of the distribution of the objects.

## Description

### Technical field

The present disclosure relates generally to a computer-implemented method for use in determining the distribution of objects in a tubular structure, in particular, a tissue construct such as an oesophageal construct. The present disclosure also relates to a corresponding apparatus, computer-readable medium and computer program.

### Background

Tissue or organ damage, dysfunction, or loss is a feature of a wide variety of medical conditions. In some such conditions, replacement of the damaged tissue or organ is the best or even the only option. For example, oesophageal atresia is a congenital medical condition which affects the alimentary tract and which occurs in approximately 1 in 2500 live births.

Transplantation from human donors (either live or cadaveric) has enjoyed significant success, and procedures such as liver, heart, and kidney transplants are becomingly increasingly common. However, this approach is limited by the severe shortage of donors, the complexity of harvesting organs and delivering them to the recipient, and the potential for transmission of infectious agents and tissue rejection.

Tissue engineering is an evolving field that seeks to develop techniques for culturing replacement tissues and organs in the laboratory.

The general strategy for producing replacement tissues utilises mammalian cells (e.g. autologous or allogeneic) that are seeded onto an appropriate substrate for cell culture. After seeding, cell growth is generally continued in the laboratory and/or in the patient following implantation of the engineered tissue.

Unfortunately, existing approaches for cell seeding and for cell culture in tissue engineering often do not result in the manufacture of a consistent product, e.g. each manufactured tissue construct may comprise a different amount of different cell populations on the scaffold. Functional tissues, particularly luminal tissue or organs, may therefore not be consistently reproducibly obtained from production methods, and there is a need to be able to assess tissue constructs to identify functional from non-functional constructs, and further to identify methods which are capable of resulting in the consistent production of a functional construct.

Although it is possible to acquire images of a scaffold and to identify the position of cells on such images, humans have difficulty reliably assessing the distribution of the cells from such images. Particularly, depth and density are often not accurately assessed by the human eye.

It would be advantageous to provide a method that addresses these problems. In particular, it would be advantageous to provide a computer-implemented method for use in determining the distribution of cells, or, more generally, the distribution of objects on a tubular structure.

### Summary

Aspects of the present disclosure are defined in the accompanying independent claims.

According to an aspect, there is provided a computer-implemented method for use in determining the distribution of objects in a tubular structure. The method comprises receiving a representation of a cross-sectional slice of the tubular structure; based on the representation, determining a number of objects in each of a plurality of circular sectors of the cross-sectional slice; and based on the number of objects in each of the plurality of circular sectors, outputting an indication of the distribution of the objects.

Optionally, the method further comprises, based on the number of objects in each of the plurality of circular sectors, determining the distribution of the objects. Determining the distribution of objects may involve determining whether any objects within a tubular structure (e.g. a portion of the objects (e.g. a particular population of objects, e.g. cells)) have a homogenous or heterogenous distribution through the structure or through a portion of the structure e.g. through the tissue construct. Thus, the homogeneity of the objects (or a portion of the objects) present may be determined by a method of the present disclosure.

Optionally, the method further comprises, based on the number of objects in each of the plurality of circular sectors, determining whether the tubular structure satisfies a distribution requirement, e.g. a homogeneity requirement.

The distribution requirement may vary depending on the tubular structure to be assessed and particularly on the type of tissue construct produced. Thus, it may be desirable in one example for the objects (e.g. cells) or for a portion of the objects (e.g. a particular cell type) present in a tissue construct to have a homogenous distribution throughout the tissue. For example, it may be desirable for a particular cell type to be regularly or evenly distributed throughout a tissue. Alternatively, it may be desirable for the objects (e.g. cells) or for a portion of the objects (e.g. a particular cell type) present in a tissue construct to have a heterogenous distribution in the tissue. For example, it may be desirable for a particular cell type to be present in a specific or particular area or position in a tissue construct (e.g. on the exposed surface of the construct, within the construct or immediately adjacent to any scaffold present).

Optionally, the method further comprises, based on the number of objects in each of the plurality of circular sectors, determining whether the tubular structure is suitable for implantation into a subject.

Optionally, the indication of the distribution of the objects is an object density map.

Optionally, the method further comprises, based on the number of objects in each of the plurality of circular sectors, determining a reference point for the object density map; and positioning the reference point in a predetermined position on the object density map by orienting the object density map.

Optionally, determining the number of the objects in each of the plurality of circular sectors comprises determining a centre of the cross-sectional slice, and wherein the plurality of circular sectors are sectors of a disk centred on the determined centre of the cross-sectional slice.

Optionally, determining the number of the objects in a respective one of the plurality of circular sectors comprises determining a number of the objects at each of a plurality of radial distances.

Optionally, for each of the plurality of circular sectors, the plurality of radial distances comprises a same number of radial distances.

Optionally, determining the number of the objects in the respective one of the plurality of circular sectors comprises,
for each respective one of the objects in the respective one of the plurality of circular sectors:
determining local inner and outer boundaries of the tubular structure for the respective one of the objects, and
assigning the respective one of the objects to one of the plurality of radial distances based on a relative position of the respective one of the objects with respect to the corresponding local inner and outer boundaries of the tubular structure; and
determining the number of the objects at each respective one of the plurality of radial distances based on how many objects have been assigned to the respective one of the plurality of radial distances.

Optionally, objects closest to their local inner boundary are assigned to the smallest of the radial distances and objects closest to their local outer boundary are assigned to the largest of the radial distances.

Optionally, determining the number of the objects in the respective one of the plurality of circular sectors comprises populating a data structure for the respective one of the plurality of circular sectors, the data structure having a plurality of data elements each corresponding to a given radial distance.

Optionally, outputting an indication of the distribution of the objects comprises outputting an object density map representing values of the data elements of each of the data structures.

Optionally, each of the objects is a biological cell or a cluster of biological cells.

Optionally, each of the objects is an extracellular matrix.

Optionally, the tubular structure is a tissue construct, for example, a construct which may be applied to tubular (e.g. luminal or hollow) organs, such as oesophagus, trachea, blood vessels, intestine, urethra, bowel etc.

Optionally, the tissue construct is an oesophageal construct.

Optionally, the tubular structure comprises a scaffold, for example an acellular scaffold such as a decellularized scaffold or a polymeric scaffold.

In a particular example, the tissue construct is an oesophageal construct comprising a scaffold. Particularly for an oesophageal construct, the objects may be selected from one or more of mesoangioblasts, fibroblasts, epithelial cells and neural crest stem cells. It may be desirable for seeded mesoangioblasts and fibroblasts in particular to have a homogenous distribution in an oesophageal construct.

According to another aspect, there is provided an apparatus arranged to perform any of the methods described herein.

According to yet another aspect, there is provided a computer-readable medium comprising computer-readable instructions which, when executed by a processor, cause the processor to carry out any of the methods described herein.

### Brief description of the drawings

Illustrative implementations of the present disclosure will now be described, by way of example only, with reference to the accompanying drawings in which:
Figure 1 shows an exemplary image of a cross-sectional slice of an oesophageal construct;
Figure 2 shows a representation of the cross-sectional slice of Figure 1;
Figures 3a and 3b illustrate the steps of a computer-implemented method for use in determining the distribution of objects on a tubular structure;
Figure 4 shows a simplified representation of a cross-sectional slice of a tubular structure;
Figure 5 shows the simplified representation of Figure 4 on which a centre of the tubular structure has been marked;
Figure 6 shows the simplified representation of Figure 5 on which a plurality of circular sectors have been identified;
Figure 7 illustrates the determination of a number of objects within one of the plurality of circular sectors;
Figure 8 illustrates the creation of an object density map;
Figures 9a, 10a, 11a and 12a illustrate representations of cross-sectional slices of a number of exemplary tubular structures and Figures 9b, 10b, 11b and 12b illustrate corresponding object density maps; and
Figure 13 illustrates a block diagram of an exemplary computing device for performing any of the methods described herein.

Throughout the description and the drawings, like reference numerals refer to like parts.

### Detailed description

In overview, the present disclosure relates to a method for analysing imagery of a tubular structure on which a number of objects of interest are present. In particular, the method involves acquiring an image of a cross-sectional slice of the structure, identifying the position of the objects in the image, splitting the image into circular sectors, determining the number of objects in each of the sectors, and outputting an indication of the distribution of the objects based on the determining.

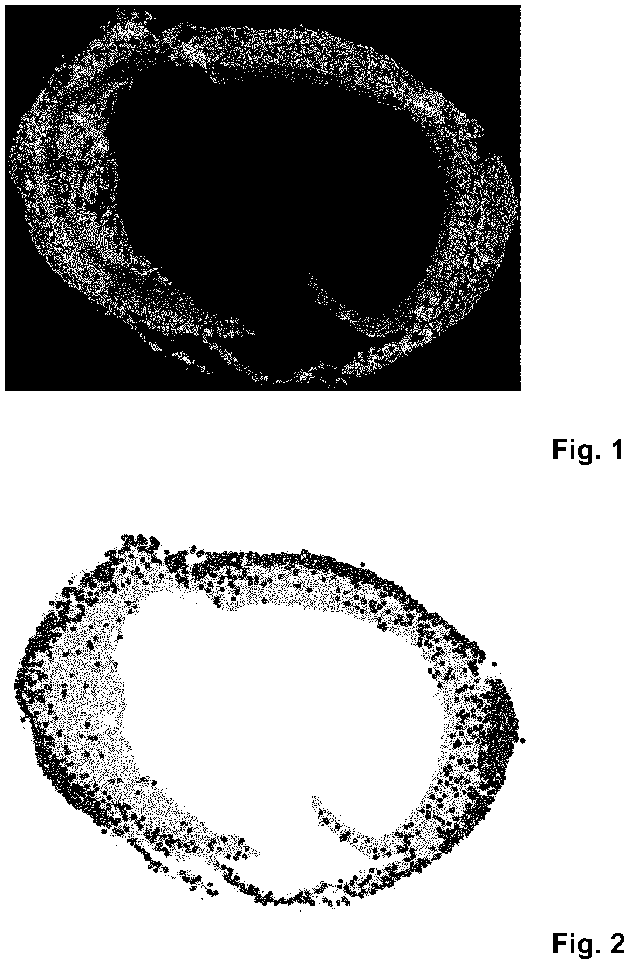

Figure 1 shows an exemplary image of a cross-sectional slice of an oesophageal construct. The image is obtained by way of fluorescence microscopy, such that cells of different types are highlighted in different colours. Using known image processing techniques, a computer can identify the position of each of the cells of a given type, so as to produce the representation shown in Figure 2, where each of the dots represents a cell on the oesophageal construct.

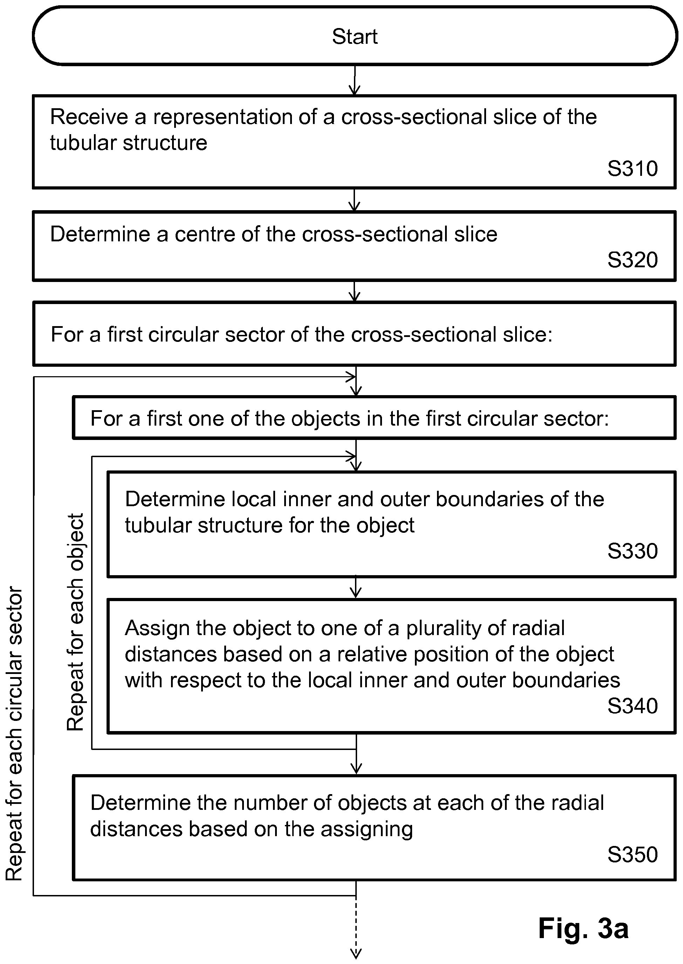

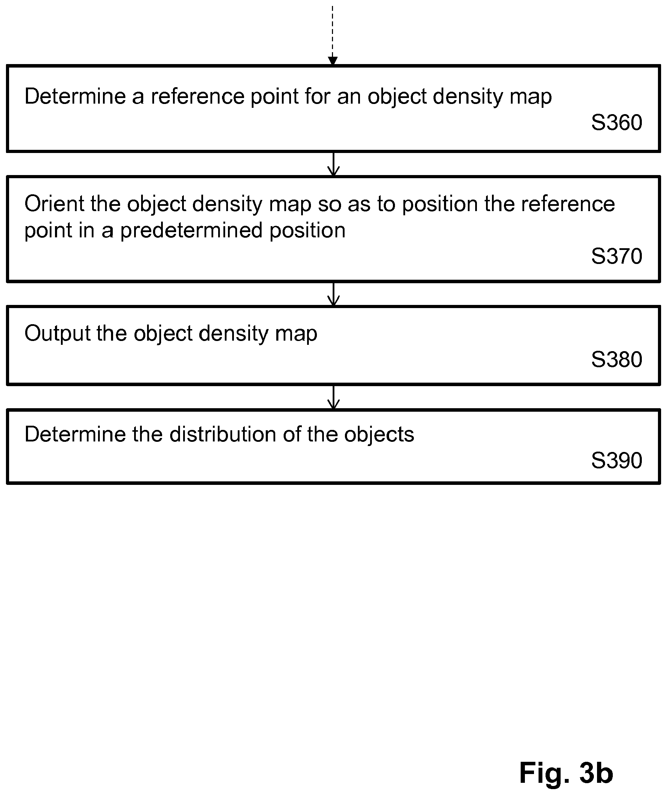

Figures 3a and 3b illustrate the steps of a computer-implemented method for use in determining the distribution of objects (or 'objects of interest') on a tubular structure, such as the distribution of cells on the oesophageal construct of Figure 2.

In step S310, a representation of a cross-sectional slice of the tubular structure is received.

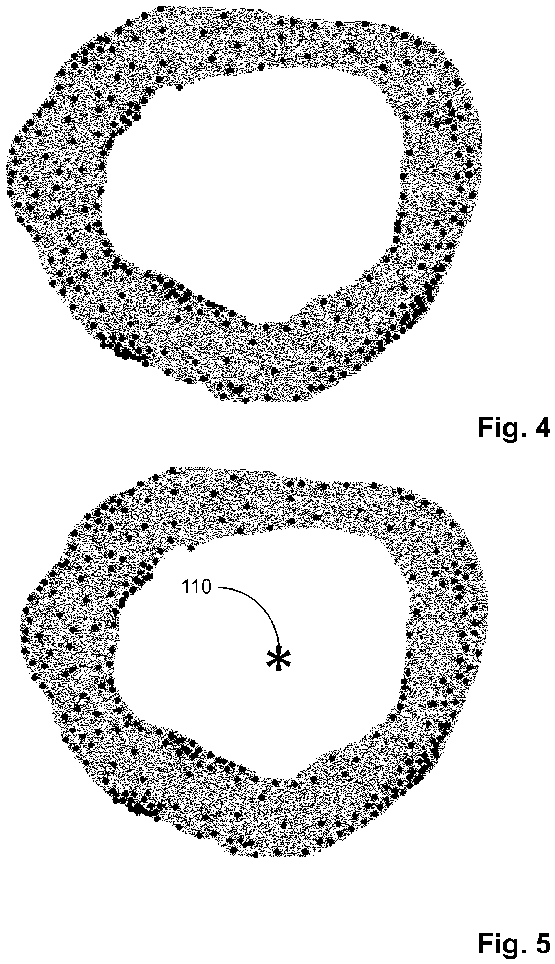

An example of such a representation is shown in simplified form in Figure 4. In Figure 4, a masking operation has been performed so as to remove unwanted features from a base image and leave only a relevant portion of the tubular structure (marked in grey). Each of the dots represents an object of interest which has been identified.

The representation forms the basis for the analysis performed in the subsequent steps of the method. Prior to the representation being received, the representation may have been processed using image processing algorithms or techniques in order to improve the performance of the below steps and/or in order to determine the positions of the objects. Receiving the representation may therefore comprise receiving an associated data structure containing the positions of the objects represented in the representation.

In step S320, a centre 110 of the cross-sectional slice is determined. The cross-section of the tubular structure is not necessarily perfectly circular, therefore the determined centre may be the centroid or geometric centre of the cross-sectional slice, or simply a centrally located point, as illustrated in Figure 5.

The determination may, in some examples, be manually performed by a user. In this case, the user may be provided with a graphical user interface via which the determined centre may be input by clicking or tapping at a particular location to identify the determined centre.

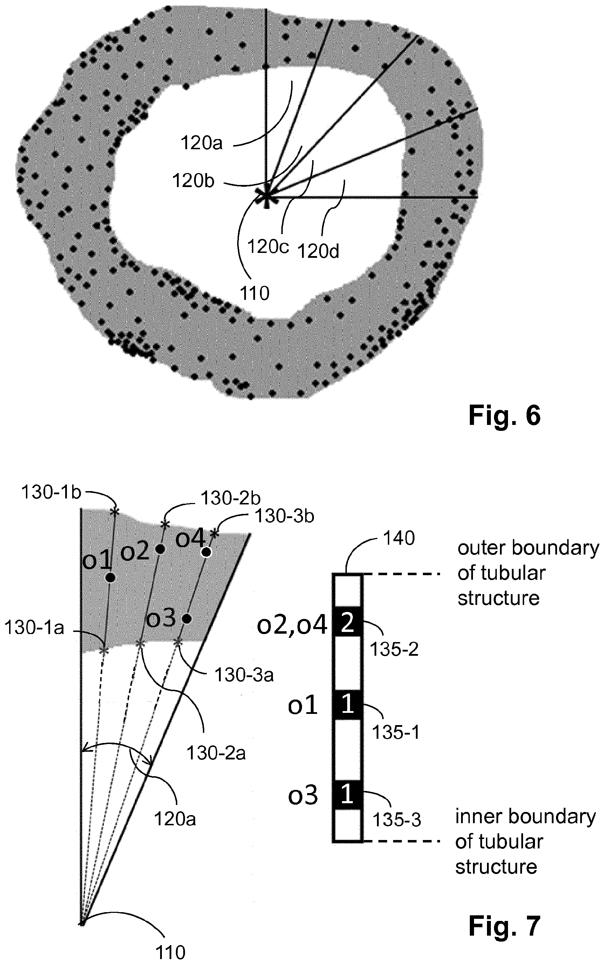

Once a centre 110 has been determined, the cross-sectional slice can notionally be split into a plurality of circular sectors 120a-120d (or 'angular sectors', 'angular sections'), as illustrated in Figure 6. A circular sector, as is known in the art, is the portion of a disk enclosed by two radii and an arc. In this case, each circular sector is a portion of a disk centred on the determined centre 110 of the cross-sectional slice. The circular sectors 120a-120d normally cover the entire disk but may, in one example, only cover a portion of the entire disk. Within each circular sector, any number of objects may be present.

The angle made by the arc may vary depending on the application. It will be understood that, the smaller the angle, the higher the resolution of the data that is determined using the method of Figures 3a and 3b. However, if the angle is too small, there may frequently be no more than one object in each circular sector, in which case the data that is determined using the method of Figures 3a and 3b may be of limited use.

The method then begins two loops: a first loop, which is executed for each of the circular sectors 120a-120d of the cross-sectional slice and, nested within the first loop, a second loop, which is executed for each of the objects. The first iteration of the first loop is for a first circular sector 120a. The first iteration of the second loop is for a first object in the first circular sector 120a.

In step S330, local inner and outer boundaries of the tubular structure for the first object in the first circular sector 120a are determined.

In particular, as illustrated in Figure 7, a line passing through the determined centre 110 of the cross-sectional slice and the position of the first object is notionally drawn, and the local inner and outer boundaries respectively correspond to the intersection of the line with the inner and outer boundaries of the tubular structure. In Figure 7, the position of those boundaries is indicated by asterisks, while the position of the objects is indicated by black circles, numbered o1 to o4.

In step S340, the object is assigned to one of a plurality of radial distances based on a relative position of the object with respect to its corresponding local inner and outer boundaries.

The radial distances may be quantised distances, in other words, each distance may in reality be a distance *range* and each object may be assigned to one of the distance *ranges.* The object may be assigned to a radial distance by assigning it to a particular position in a column vector.

Objects closest to their corresponding local inner boundary are assigned to the smallest of the radial distances and objects closest to their corresponding local outer boundary are assigned to the largest of the radial distances.

Steps S330-S340 are then repeated for each of the remaining objects in the first circular sector 120a.

In the example of Figure 7, object 01 is approximately halfway between its corresponding local inner and outer boundaries, respectively represented by asterisks 130-1a and 130-1b, and is therefore assigned to a position 135-1 halfway along the column vector 140. Object o2 is closer to its corresponding local outer boundary, represented by asterisk 130-2b, than its corresponding local inner boundary, represented by asterisk 130-2a, and so object o2 is assigned to a position 135-2 closer to a top end of the column vector 140. Object o3 is located closer to its corresponding local inner boundary, represented by asterisk 130-3a, than its corresponding local outer boundary, represented by asterisk 130-3b, and so object o3 is assigned to a position 135-3 closer to a bottom end of the column vector 140. Although object o4 is located slightly closer to its corresponding local outer boundary 130-3b than object o2 is to its corresponding local outer boundary 130-2b, the *relative* position of object o4 with respect to its corresponding local inner and outer boundaries 130-3a and 130-3b is similar to the relative position of object o2 with respect to its corresponding local inner and outer boundaries 130-2a and 130-2b, and therefore object o4 is assigned to the same position 135-2 of the column vector 140 as object o2.

In step S350, the number of objects at each of the plurality of radial distances is determined. The determining is based on the assigning. In particular, the determining may be based on how many objects have been assigned to each of the plurality of radial distances.

In the example of Figure 7, one object has been assigned to each of positions 135-1 and 135-3, and two objects have been assigned to position 135-2. These values are recorded in the column vector 140.

In performing steps S340-S350, a data structure may be populated for the first circular sector 120a. The data structure has a plurality of data elements each corresponding to a given radial distance. Examples of such data structures include arrays and vectors.

The data structure may initially hold zero values in all data elements. Subsequently, when an object is assigned to a particular radial distance, the value of the corresponding data element may then be incremented.

Steps S330-S350 are then repeated for each of the remaining circular sectors 120b-120d. A number of the objects in each of a plurality of circular sectors 120a-120d of the cross-sectional slice is thereby determined. In so doing, a data structure 140 may be populated for each of the plurality of circular sectors 120a-120d.

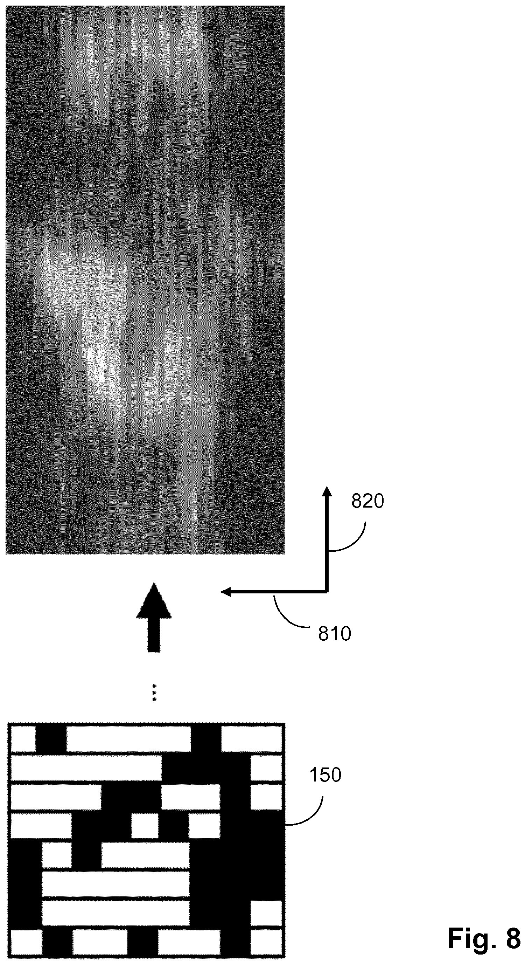

It will be appreciated that each of those data structures 140 may form part of a larger data structure 150, such as a matrix or table, as illustrated in Figure 8. In this larger structure, the smaller structures 140 may be linked so as to form an ordered sequence.

In one example, the number of radial distances is the same for each of the plurality of circular sectors 120a-120d. When column vectors are used to hold the determined numbers, each of the column vectors may thus have the same length.

The distribution of the objects, e.g. the homogeneity of the distribution of the objects may then be determined in various ways, depending on the application. These various ways have in common that an indication of the distribution of the objects is output.

In one example, an object density map (or 'oriented object density map', or 'object distribution map') is created or generated. This map represents the number of objects in each of the plurality of circular sectors 120a-120d and at each of the radial distances. In examples where a data structure is populated for each of the plurality of circular sectors 120a-120d, or a larger data structure is populated for the plurality of circular sectors 120a-120d, the map may represent values of the data elements of each of the data structures, or of the larger data structure.

The values of the data elements may be mapped to a colour by means of a colour map. In one example, lighter colours are used for higher values, and darker colours are used for lower values.

An illustration of the creation of an object density map is provided in Figure 8. At the bottom of the figure, a series of column vectors are shown. Elements of the vectors having non-zero values are marked in black. At the top of the figure, a corresponding object density map is shown. A first axis 810 of the figure indicates the radial distance, while the second axis 820 indicates the circular sector (or equivalently, the polar angle).

In order for the object density maps for a varied set of representations of tubular structures to be interpreted or analysed in a consistent way, it may be useful for the object density maps to be oriented in a consistent way. For example, when comparing three consecutive tissue sections with expected similar object distributions, experimental procedures for analysing the tissue sections may orient these sections randomly, thus complicating the analysis. The present approach may avoid this issue.

Accordingly, in step S360, a reference point for the object density map is determined. The reference point may, for example, be a point having the highest number of objects associated therewith, a point having the lowest number of objects associated therewith, a point having no objects associated therewith, a central point of an area having the highest number of objects associated therewith, a central point of an area having the lowest number of objects associated therewith, or a central point of an area having no objects associated therewith.

As the position of the point having the *highest* number of objects associated therewith can vary significantly between density maps computed from consecutive tissue sections, while the overall distribution of objects remains the same, it may be preferable for the reference point to instead be based on the point having the *lowest* number of objects associated therewith.

The point having the lowest number of objects associated therewith may be determined by determining a sum or average, over the plurality of radial distances, of the number of objects in each of the circular sectors, and smoothing the resulting curve of sums/averages to remove *local* minima, thereby emphasising a *global* minimum instead. In the event that a single global minimum cannot be determined, e.g., when a large portion of the tissue does not contain any objects at all, the reference point may be determined as a point that is equidistant from the first circular sectors that *do* contain objects on either side of this point. In other words, a point located in the middle of an empty area of the density map, as determined after the sums/averages have been determined and the smoothing has been performed.

In step S370, the object density map is oriented (or 'aligned', or 'rotated', or 'shifted') so as to position the reference point in a predetermined position. For example, the reference point may be positioned in the centre of the object density map, or at one of the corners of the object density map.

The object density map may additionally or alternatively be oriented such that the inner portion of the tubular structure (the lumen) is positioned at the bottom of the map and the outer portion of the tubular structure is positioned at the top of the map, or vice versa.

In step S380, the object density map is output. The map may be output via any suitable output means, such as a display or monitor or a printer.

In step S390, the distribution of objects is determined. The determining is based on the number of the objects in each of the plurality of circular sectors 120a-120d. Additionally or alternatively, it may be determined whether the tubular structure is suitable for implantation into a subject or patient, whether the tubular structure satisfies a homogeneity requirement, whether the tubular structure has been produced/manufactured to acceptable standards, whether manufacturing processes used to manufacture the tubular structure are reproducible, and/or whether the tubular structure is suitable for clinical applications. The determination(s) may, in some examples, be manually performed by a user.

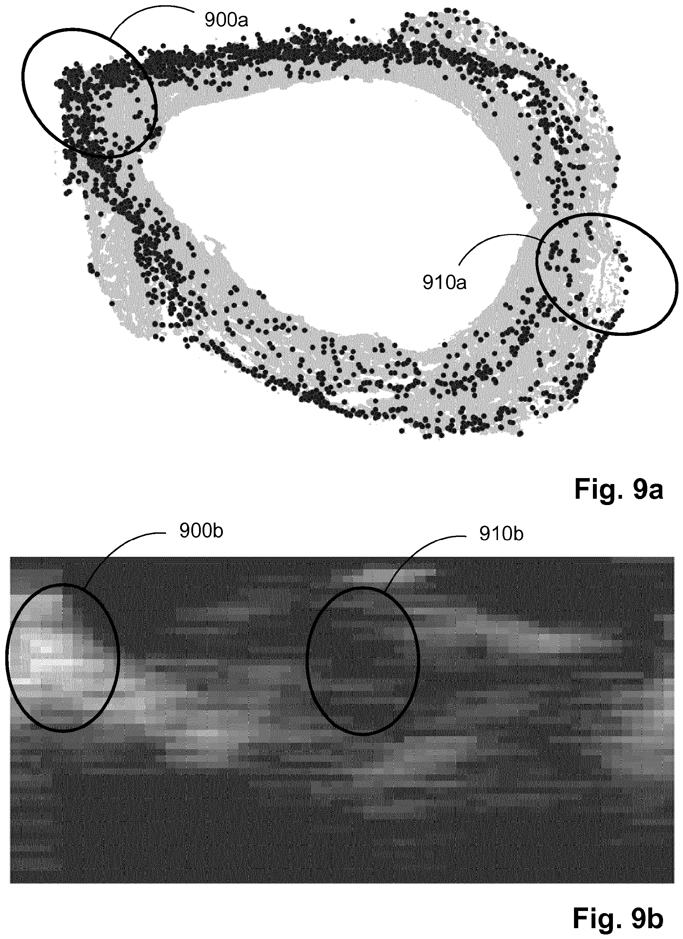

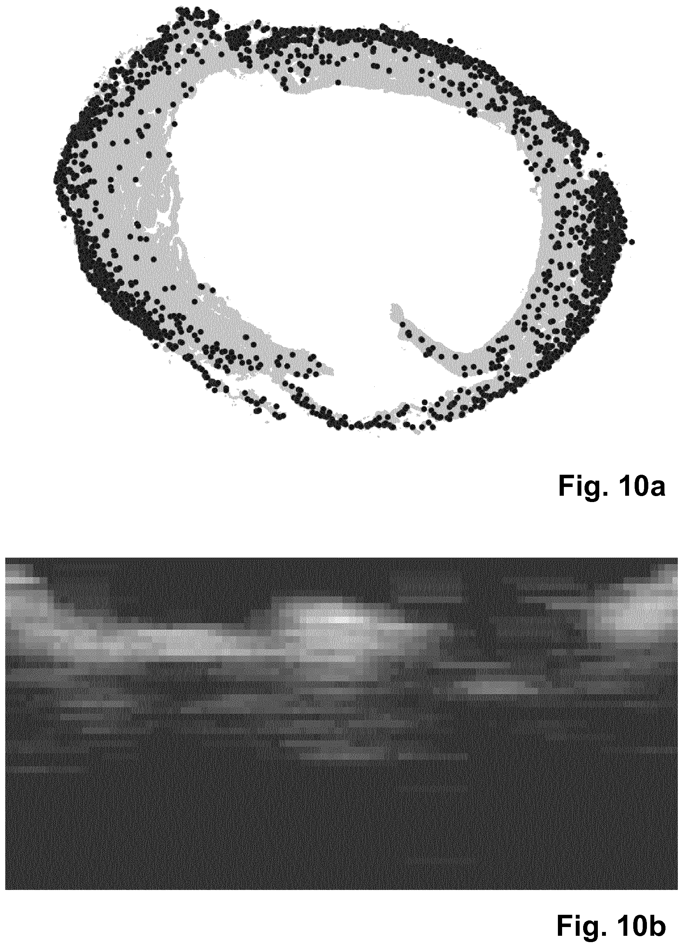

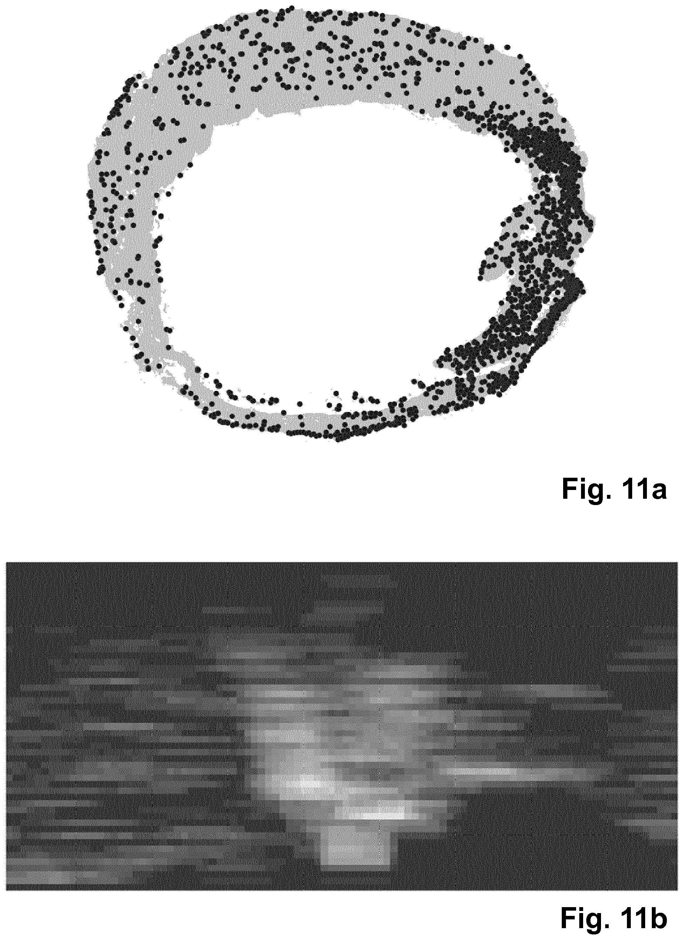

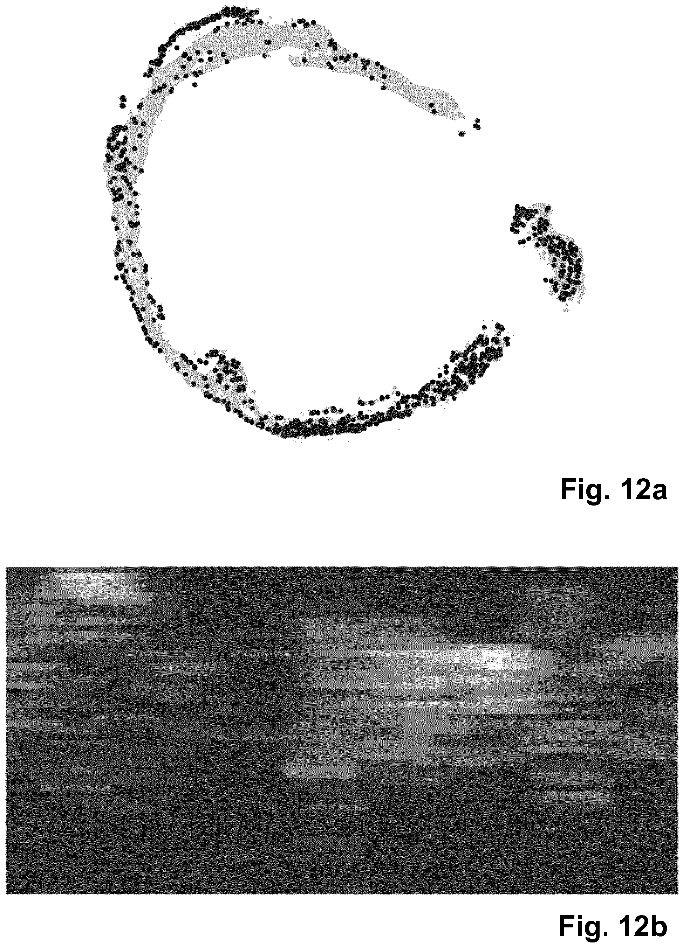

Figures 9a, 10a, 11a and 12a illustrate representations of cross-sectional slices of a number of exemplary tubular structures and Figures 9b, 10b, 11b and 12b illustrate the corresponding object density maps.

In Figure 9a, it can be seen that there is a high density of objects in area 900a, and a low density of objects in area 910a. The area of high density 900a corresponds to area 900b in Figure 9b, while the area of low density 910a corresponds to area 910b in Figure 9b. Area 900b is shown in a generally lighter colour than area 910b to reflect the higher density of objects in the former area.

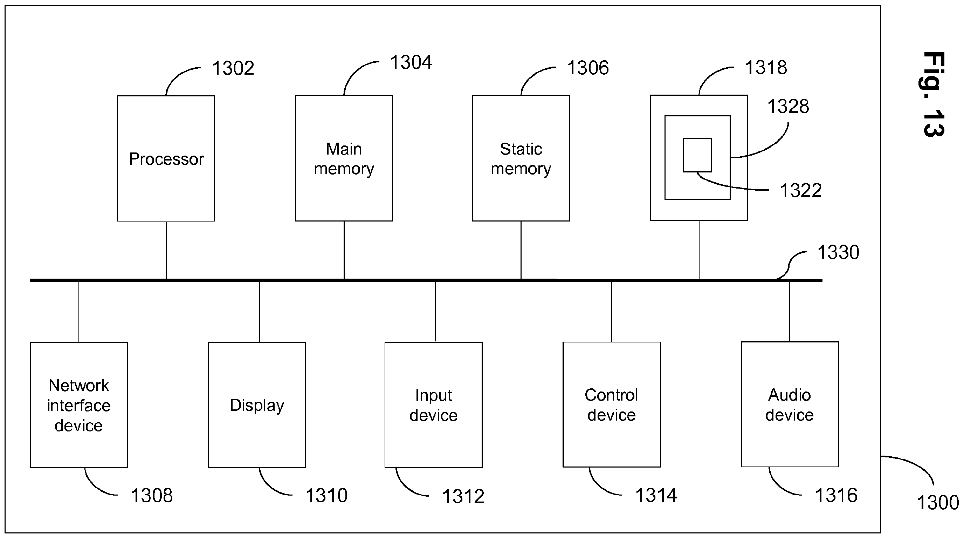

Figure 13 illustrates a block diagram of one implementation of a computing device 1300 within which a set of instructions, for causing the computing device to perform any one or more of the methodologies discussed herein, may be executed. In alternative implementations, the computing device may be connected (e.g., networked) to other machines in a Local Area Network (LAN), an intranet, an extranet, or the Internet. The computing device may operate in the capacity of a server or a client machine in a client-server network environment, or as a peer machine in a peer-to-peer (or distributed) network environment. The computing device may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine. Further, while only a single computing device is illustrated, the term "computing device" shall also be taken to include any collection of machines (e.g., computers) that individually or jointly execute a set (or multiple sets) of instructions to perform any one or more of the methodologies discussed herein.

The example computing device 1300 includes a processing device 1302, a main memory 1304 (e.g., read-only memory (ROM), flash memory, dynamic random access memory (DRAM) such as synchronous DRAM (SDRAM) or Rambus DRAM (RDRAM), etc.), a static memory 1306 (e.g., flash memory, static random access memory (SRAM), etc.), and a secondary memory (e.g., a data storage device 1318), which communicate with each other via a bus 1330.

Processing device 1302 represents one or more general-purpose processors such as a microprocessor, central processing unit, or the like. More particularly, the processing device 1302 may be a complex instruction set computing (CISC) microprocessor, reduced instruction set computing (RISC) microprocessor, very long instruction word (VLIW) microprocessor, processor implementing other instruction sets, or processors implementing a combination of instruction sets. Processing device 1302 may also be one or more special-purpose processing devices such as an application specific integrated circuit (ASIC), a field programmable gate array (FPGA), a digital signal processor (DSP), network processor, or the like. Processing device 1302 is configured to execute the processing logic (instructions 1322) for performing the operations and steps discussed herein.

The computing device 1300 may further include a network interface device 1308. The computing device 1300 also may include a video display unit 1310 (e.g., a liquid crystal display (LCD) or a cathode ray tube (CRT)), an alphanumeric input device 1312 (e.g., a keyboard or touchscreen), a cursor control device 1314 (e.g., a mouse or touchscreen), and an audio device 1316 (e.g., a speaker).

The data storage device 1318 may include one or more machine-readable storage media (or more specifically one or more non-transitory computer-readable storage media) 1328 on which is stored one or more sets of instructions 1322 embodying any one or more of the methodologies or functions described herein. The instructions 1322 may also reside, completely or at least partially, within the main memory 1304 and/or within the processing device 1302 during execution thereof by the computer system 1300, the main memory 1304 and the processing device 1302 also constituting computer-readable storage media.

The various methods described above may be implemented by a computer program. The computer program may include computer code arranged to instruct a computer to perform the functions of one or more of the various methods described above. The computer program and/or the code for performing such methods may be provided to an apparatus, such as a computer, on one or more computer-readable media or, more generally, a computer program product. The computer-readable media may be transitory or non-transitory. The one or more computer-readable media could be, for example, an electronic, magnetic, optical, electromagnetic, infrared, or semiconductor system, or a propagation medium for data transmission, for example for downloading the code over the Internet. Alternatively, the one or more computer-readable media could take the form of one or more physical computer-readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may be or include a special-purpose processor, such as a field programmable gate array (FPGA) or an ASIC. A hardware component may also include programmable logic or circuitry that is temporarily configured by software to perform certain operations.

Accordingly, the phrase "hardware component" should be understood to encompass a tangible entity that may be physically constructed, permanently configured (e.g., hardwired), or temporarily configured (e.g., programmed) to operate in a certain manner or to perform certain operations described herein.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

Although the present disclosure has been set out in the context of oesophageal constructs, the tubular structure could be any structure having a tubular or luminal shape, such as a trachea construct, an intestinal construct, or any other tissue construct. In another example, the tubular structure could form part of a transplanted tissue, rather than a grown tissue.

Although the present disclosure describes the analysis of biological cells, the present approaches apply equally to other objects such as clusters of biological cells, extracellular molecules, extracellular matrices, or gaps in endothelial cells.

It will be understood that the ordering of the steps in the method of Figures 3a and 3b is merely exemplary, and that the steps could be performed in a different order. In particular, steps S330 and/or S340 could be performed for objects in multiple circular sectors, with step S350 subsequently being performed on a sector-by-sector basis.

It will also be understood that the steps of Figures 3a and 3b need not all be performed and that, unless otherwise indicated, these steps are optional. In particular, steps S360-S390 need not be performed.

An effect of the present disclosure is the provision of an objective approach for evaluating or characterising a tubular structure. Particularly, where the tubular structure is a tissue construct, an effect of the present disclosure may be the determination of whether the construct is likely to be functional or effective (e.g. when transplanted into a patient), which may be assessed based on the distribution of particular objects within the construct (e.g. mesoangioblasts, and fibroblasts). "Functional" or "effective" may refer to a construct which has at least partial activity as compared to an equivalent naturally occurring tissue in a subject (e.g. at least 20, 30, 40, 50, 60, 70, 80 or 90% activity) or which is able to improve or restore the function of a damaged or absent tissue in a subject.

Further, an effect of the present disclosure may be the identification of a method for producing a tubular structure which consistently provides a tubular structure with a desired distribution of objects (and which may therefore be a functional, effective or desirable tubular structure, e.g. tissue construct). For a tubular structure having a desired distribution of objects to be consistently produced, it may be produced from a method with an occurrence of at least 70, 80, 90, 95 or 99%. Particularly, a production method may be identified which always produces a tubular structure having a desired distribution of objects.

An effect of the present disclosure is that very small objects can be detected in comparatively very large areas.

When the angle of each circular sector is small, the area covered by a given circular sector and a given radial distance may be almost rectangular. An effect thereof is that visualisation distortions in the resulting map may be reduced.

Unless specifically stated otherwise, it is appreciated that throughout the description, discussions utilizing terms such as "receiving", "determining", "assigning", "outputting", "positioning", "identifying", "orienting" or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

The singular terms "a" and "an" should not be taken to mean "one and only one". Rather, they should be taken to mean "at least one" or "one or more" unless stated otherwise.

The word "comprising" and its derivatives including "comprises" and "comprise" include each of the stated features, but does not exclude the inclusion of one or more further features.

It is to be understood that the above description is intended to be illustrative, and not restrictive. It will be appreciated that variations of the described implementations may be made without departing from the scope of the invention.

## Claims

1. A computer-implemented method for use in determining the distribution of objects in a tubular structure, the method comprising:
receiving a representation of a cross-sectional slice of the tubular structure;
based on the representation, determining a number of objects in each of a plurality of circular sectors of the cross-sectional slice; and
based on the number of objects in each of the plurality of circular sectors, outputting an indication of the distribution of the objects.

2. The method of claim 1, further comprising:
based on the number and/or type of objects in each of the plurality of circular sectors, determining the distribution of the objects.

3. The method of any preceding claim, further comprising:
based on the number of objects in each of the plurality of circular sectors, determining whether the tubular structure satisfies a homogeneity requirement.

4. The method of any preceding claim, further comprising:
based on the number of objects in each of the plurality of circular sectors, determining whether the tubular structure is suitable for implantation into a subject.

5. The method of any preceding claim, wherein the indication of the distribution of objects is an object density map;
and optionally wherein the method further comprises:
based on the number of the objects in each of the plurality of circular sectors, determining a reference point for the object density map; and
positioning the reference point in a predetermined position on the object density map by orienting the object density map.

6. The method of any preceding claim, wherein determining the number of objects in each of the plurality of circular sectors comprises determining a centre of the cross-sectional slice, and wherein the plurality of circular sectors are sectors of a disk centred on the determined centre of the cross-sectional slice.

7. The method of any preceding claim, wherein determining the number of the objects in a respective one of the plurality of circular sectors comprises determining a number of the objects at each of a plurality of radial distances.

8. The method of claim 7, wherein, for each of the plurality of circular sectors, the plurality of radial distances comprises a same number of radial distances.

9. The method of any of claims 7 to 8, wherein determining the number of the objects in the respective one of the plurality of circular sectors comprises,
for each respective one of the objects in the respective one of the plurality of circular sectors:
determining local inner and outer boundaries of the tubular structure for the respective one of the objects, and
assigning the respective one of the objects to one of the plurality of radial distances based on a relative position of the respective one of the objects with respect to the corresponding local inner and outer boundaries of the tubular structure; and
determining the number of the objects at each respective one of the plurality of radial distances based on how many objects have been assigned to the respective one of the plurality of radial distances.

10. The method of claim 9, wherein objects closest to their local inner boundary are assigned to the smallest of the radial distances and objects closest to their local outer boundary are assigned to the largest of the radial distances.

11. The method of any of claims 7 to 10, wherein determining the number of the objects in the respective one of the plurality of circular sectors comprises populating a data structure for the respective one of the plurality of circular sectors, the data structure having a plurality of data elements each corresponding to a given radial distance;
and optionally wherein outputting an indication of the distribution of the objects comprises outputting an object density map representing values of the data elements of each of the data structures.

12. The method of any preceding claim,
wherein each of the objects is a biological cell or a cluster of biological cells; or
wherein each of the objects is an extracellular matrix.

13. The method of any preceding claim, wherein the tubular structure is a tissue construct;
and optionally wherein the tissue construct is an oesophageal construct.

14. The method of any preceding claim, wherein the tubular structure comprises a scaffold.

15. An apparatus arranged to perform the method of any preceding claim or a computer-readable medium comprising computer-readable instructions which, when executed by a processor, cause the processor to carry out the method of any of preceding claim.
